# EUROPÄISCHE PATENTANMELDUNG

(11) **EP 0 860 179 A2**
(43) Veröffentlichungstag der Anmeldung: **26.08.1998**
(21) Anmeldenummer: 97122583.4
(22) Anmeldetag: 20.12.1997
(51) Int. Cl.: A61N 5/06

(54) **Vorrichtung zur therapeutischen oder kosmetischen Behandlung mit Licht**

(30) Priorität: 21.02.1997 DE 29703132 U
(71) Anmelder: Schipke, Ulf, 93149 Nittenau (DE)
(72) Erfinder: Schipke, Ulf, 93149 Nittenau (DE)
(74) Vertreter: Graf, Helmut, Dipl.-Ing.

(57) **Zusammenfassung**

Die Erfindung bezieht sich auf eine neuartige Vorrichtung zur Magnetfeld- und/oder Strahlungstherapie und/oder zur therapeutischen oder kosmetischen Behandlung mit Licht.

## Beschreibung

Vorrichtungen zur therapeutischen oder kosmetischen Behandlung mit Licht sind bekannt (auch als Sonnenliegen). Sie bestehen aus einem Untergestell, welches eine Liegefläche bildet, und aus einem über dem Untergestell angeordneten Lichthimmel, der die Lampen für das therapeutische oder kosmetische Licht enthält.

Aufgabe der Erfindung ist es, eine Vorrichtung aufzuzeigen, die eine besonders wirksame Lichtbehandlung, aber auch Strahlungsbehandlung (beispielsweise mit Infrarotlicht) oder Magnetfeldtherapie ermöglicht. Zur Lösung dieser Aufgabe ist eine Vorrichtung entsprechend dem Schutzanspruch 1 ausgebildet.

Bei der Erfindung ist ein Teil der Liegefläche als Schwingfläche ausgebildet, der durch einen Vibrator eine dreidimensionale Schwingung aufgeprägt wird. Durch die Kombination dieser Schwingbewegung und des verwendeten Lichtes, Magnetfeldes und/oder Strahlung (z.B. Infrarotstrahlung) wird als Synergieeffekt ein besonders wirksames Behandlungsergebnis erreicht.

Weiterbildungen sind Gegenstand der Unteransprüche.

Die Erfindung wird im Folgenden anhand der Figuren an einem Ausführungsbeispiel näher erläutert. Es zeigen:
Fig. 1 in perspektivischer Darstellung eine Vorrichtung gemäß der Erfindung;

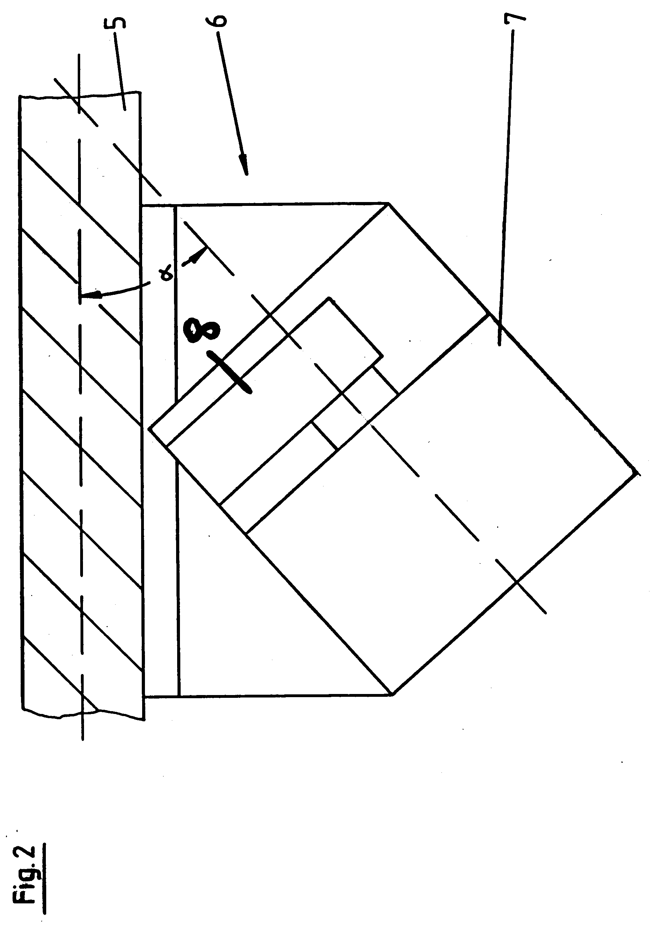

Fig. 2 in sehr vereinfachter Darstellung einen Schnitt durch die schwingende Auflage (Schwingfläche) und den an der Unterseite dieser Schwingfläche vorgesehenen mechanischen Schwingungserzeuger.

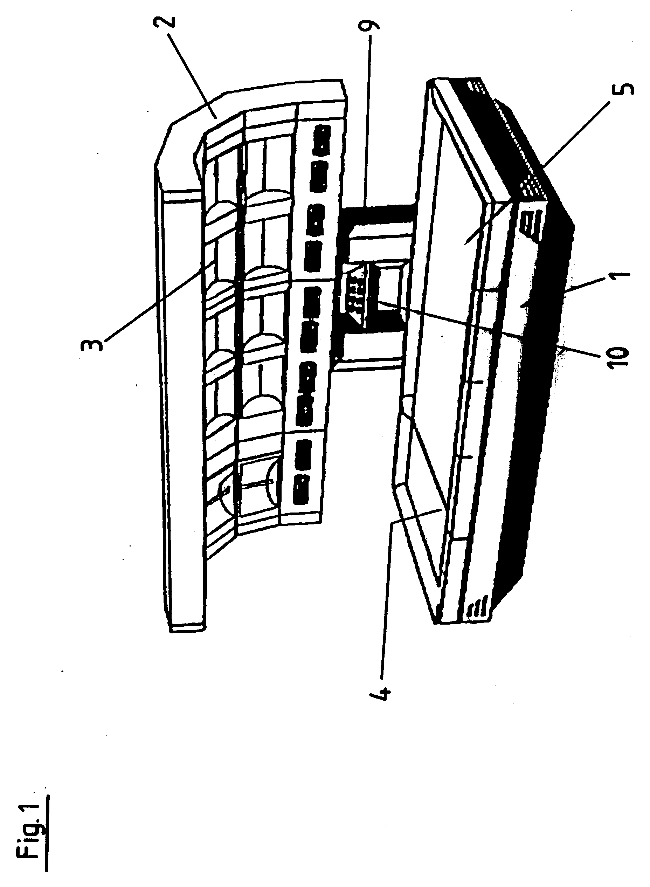

Die in der Figur 1 wiedergegebene Vorrichtung besteht aus einem Untergestell 1 und aus einem über diesem Untergestell angeordneten, verstellbaren Lichthimmel 2, der in von Sonnenliegen bekannter Weise an der Unterseite konkav gewölbt ist und dort die für die Behandlung notwendigen Lampen 3 mit Reflektoren usw. aufweist.

Das Untergestell bildet unterhalb des Lichthimmels 2 eine Liegefläche für den jeweiligen Benutzer oder die jeweils zu behandelnde Person. Die Liegefläche ist bei der dargestellten Ausführungsform unterteilt in eine kürze beruhigte, d.h. nicht bewegte Kopfauflage 4 und eine größere oder längere, die Auflage für den übrigen Körper des Benutzers bildende Schwingfläche 5. Die Schwingfläche 5 ist am Untergestell beispielsweise mit Hilfe von federnden oder elastischen Lagern derart gelagert, daß eine Schwingbewegung sowohl in den die Ebene der Schwingfläche 5 definierenden und senkrecht zueinander verlaufenden Raumachsen, als auch in der hierzu senkrecht verlaufenden Raumachse, d.h. in der Raumachse senkrecht zur Ebene der Schwingfläche 5 möglich ist. Zur Erzeugung dieser dreidimensionalen Schwingbewegung ist an der Unterseite der Schwingfläche 5 ein Vibrator 6 vorgesehen, der im wesentlichen aus einem Elektromotor 7 mit einer an der Welle des Motors exzentrisch vorgesehenen Masse 8 besteht. Zur Erzielung der dreidimensionalen Bewegung ist die Achse des Motors 6 gegenüber der Ebene der Schwingfläche 5 geneigt, d.h. diese Achse schließt mit der Ebene der Schwingfläche Seinen Winkel α < 90°, beispielsweise einen Winkel von 45° ein. Bei der dargestellten Ausführungsform ist der Motor 7 so angeordnet, daß der Winkel α zwischen der Achse des Motors und der sich in Längsrichtung der rechteckförmigen Schwingfläche 5 erstreckenden Mittelachse gebildet ist.

Der Lichthimmel 2 ist in üblicher Weise über einen seitlichen Halterahmen 9 mit dem Unterteil verbunden. An diesem Halterahmen befinden sich dann die Bedienungselemente 10, insbesondere auch solche, mit denen eine Steuerung des Vibrators 6 möglich ist, d.h. daß Ein- und Ausschalten des Vibrators sowie evtl. eine Änderung der Drehgeschwindigkeit des Motors 7.

Grundsätzlich besteht auch die Möglichkeit, anstelle der Lampen 3 oder zusätzlich zu diesen am Lichthimmel oder an einem anderen geeigneten Ort, beispielsweise unterhalb der Schwingfläche 5 führt ein Magnetfeld-Therapie wenigstens eine Quelle zur Erzeugung eines Magnetfeldes vorzusehen. Weiterhin besteht die Möglichkeit, anstelle der Lampen 3 oder zusätzlich zu diesen am Lichthimmel oder an einem anderen geeigneten Ort wenigstens eine Quelle zur Erzeugung einer Strahlung, beispielsweise einer Infrarot-Strahlung.

Weiterhin ist es auch möglich, die Kopfauflage 4 gefedert am Untergestell vorzusehen.

## Patentansprüche

1. Vorrichtung zur Magnetfeld- und/oder Strahlungstherapie und/oder zur therapeutischen oder kosmetischen Behandlung mit Licht, **gekennzeichnet** durch eine an einem Unterteil (1) der Vorrichtung schwingend gelagerte Schwingfläche (5), die eine Auflagefläche für wenigstens einen Teil des Körpers einer zu behandelnden Person bildet, durch einen an der Schwingfläche (5) vorgesehenen Vibrator (6) zur Erzeugung einer dreidimensionalen Schwingung für die Schwingfläche (5) mit Schwingungskomponenten sowohl in der Ebene der Schwingfläche als auch quer oder senkrecht zu dieser Ebene, sowie mit wenigstens einer sich mit der Schwingfläche nicht mitbewegten Einrichtung (2, 3) zur Erzeugung einer therapeutischem oder kosmetischen Lichtes und/oder eines Magnetfeldes und/oder einer Energiestrahlung.

2. Vorrichtung nach Anspruch 1, dadurch gekennzeichnet, daß über der Schwingfläche (5) eine Halterung, beispielsweise ein Lichthimmel (2) mit wenigstens einer Lampe (3) zur Erzeugung des therapeutischen und/oder kosmetischen Lichts vorgesehen ist.

3. Vorrichtung nach Anspruch 1 oder 2, dadurch gekennzeichnet, daß über der Schwingfläche (5) an einem Halter (2) wenigstens eine Infrarot-Strahlungsquelle vorgesehen ist.

4. Vorrichtung nach einem der vorhergehenden Ansprüche, dadurch gekennzeichnet, daß der Vibrator (6) von einem Elektromotor mit einer exzentrischen Masse an der Motorwelle gebildet ist, und daß die Motorwelle einen Winkel (α), beispielsweise einen Winkel von 45° mit der Ebene der Schwingfläche (5) einschließt.
